# EUROPEAN PATENT APPLICATION

(11) **EP 4 108 295 A1**
(43) Date of publication of application: **28.12.2022**
(21) Application number: 22176479.8
(22) Date of filing: 31.05.2022
(51) Int. Cl.: A61N 2/06, A01K 1/015

(54) **MAT FOR ANIMALS**

(30) Priority: 22.06.2021 IT 202100016253
(71) Applicant: Pet Creation Srl, 31011 Asolo (TV) (IT)
(72) Inventor: PAOLIN, Maria Beatrice, 31011 ASOLO TV (IT)
(74) Representative: Modiano, Micaela Nadia

(57) **Abstract**

A mat (10) for animals, which comprises at least the following layers:
- at least one first fabric (11) containing silver fiber,
- at least one second fabric (12) containing copper fiber,
- at least one padding (13), which contains heat-reactive and/or photocatalytic minerals and is sandwiched between the first fabric (11) and the second fabric (12).

The mat (10) also comprises magnetic bands (14), which are arranged according to a same orientation, are mutually spaced and are interposed between the at least one padding (13) and the at least one second fabric (12).

## Description

The present invention relates to a mat for animals.

Various kinds of mats for animals, particularly but not exclusively for pets such as dogs, intended to facilitate their physical wellbeing, are currently commercially available.

For example, there are heated kennels, the purpose of which is to keep the animal warm, or mats the operation of which is based on well-known magnetotherapy, which uses the effects that magnetic fields produce in the body to obtain an anti-inflammatory and antalgic action, particularly to treat osteoarticular pain, or even just to obtain a relaxing effect.

It is known in the literature that static magnetotherapy activates microcirculation to combat various inflammatory pathologies such as arthrosis, which has now become a common pathology of pets, such as dogs, cats and horses. The chronic and degenerative process of cartilages and subsequently also of muscle mass and posture causes animals, over time, to have motor difficulty and continuous pain. It appears that magnetotherapy improves the oxygen levels in the skin, tissues and muscles, accelerating healing from inflammatory processes, and that by acting also at the bone level it contrasts osteoporosis and accelerates fracture healing processes. It appears to be useful also in the case of tendinitises, contusions, sprains, strains, rheumatic diseases or articular pathologies causing pain, since it tends to limit inflammatory processes.

The use of magnetotherapy mats is not invasive and is found to be effective in the treatment of motor disorders and pain.

These mats are often crossed by an electrical cord to be connected to an electric current outlet or to a battery, in order to generate a magnetic field which acts on the body of the animal.

They have a series of drawbacks, due to the fact that they need to charge the battery or require the availability of a nearby power outlet, in addition to the need to use a chew-resistant electrical cord for the safety of the animal.

Furthermore, since they are connected to an electrical outlet, they are a source of electromagnetic wave pollution.

The aim of the present invention is to provide a mat for animals that is capable of improving the background art in one or more of the aspects indicated above.

Within this aim, an object of the invention is to provide a mat that utilizes the principles of magnetotherapy, without using electrical cords, in order to improve and prevent some pathologies of animals, such as muscular, articular, skeletal problems.

Another object of the invention is to provide a mat the properties of which remain unchanged over time.

A further object of the invention is to provide a mat for animals that is smellreducing, antibacterial, antistatic and antimicrobial.

A still further object of the present invention is to overcome the drawbacks of the background art in a manner that is alternative to any existing solutions.

This aim, as well as these and other objects which will become better apparent hereinafter, are achieved by a mat for animals, characterized in that it comprises at least the following layers:
- at least one first fabric containing silver fiber,
- at least one second fabric containing copper fiber,
- at least one padding, which contains heat-reactive and/or photocatalytic minerals and is sandwiched between said first fabric and said second fabric,
said mat also comprising magnetic bands, which are arranged according to a same orientation, are mutually spaced and are interposed between said at least one padding and said at least one second fabric.

Further characteristics and advantages of the invention will become better apparent from the description of a preferred but not exclusive embodiment of the mat according to the invention, illustrated by way of non-limiting example in the accompanying drawings, wherein:
Figure 1 is a perspective view of the mat according to the invention;
Figure 2 is a top view of the mat according to the invention;

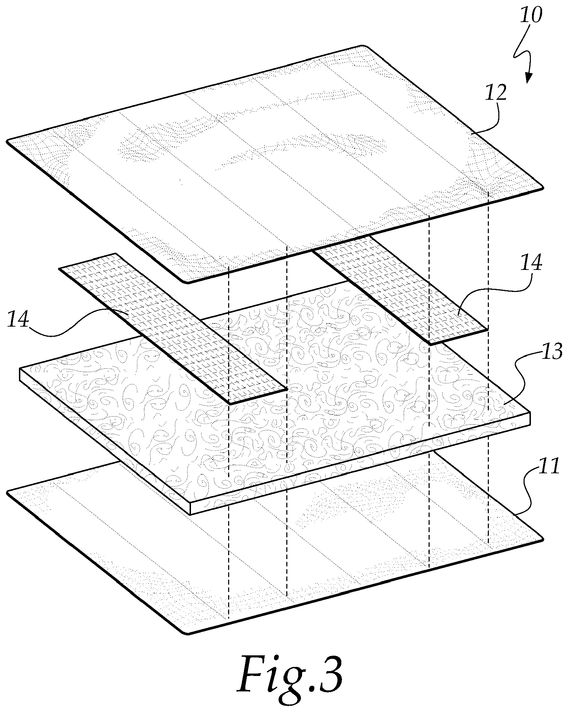

Figure 3 is an exploded perspective view of the mat according to the invention.

With reference to the figures, the mat according to the invention, generally designated by the reference numeral 10, comprises the following layers:
- at least one first fabric 11 containing silver fiber,
- at least one second fabric 12 containing copper fiber,
- at least one padding 13, which contains heat-reactive and/or photocatalytic minerals, which is sandwiched between the first fabric 11 and the second fabric 12.

Advantageously, the mat 10 also comprises magnetic bands 14, which are arranged along a same orientation, are mutually spaced and are interposed between the padding 13 and the second fabric 12.

The magnetic bands 14 are flexible, with a flattened and elongated shape, and contain permanent magnets, embedding powders of magnetite, ferrite, strontium and neodymium.

The distance between the magnetic bands 14 is preferably approximately equal to twice their width. Here the term "width" refers to the dimension of the shorter side, the term "length" refers to the dimension of the longer side, and the term "height" refers to the dimension of thickness, which is such as to produce said flattened shape.

The padding 13 is of the Celliant^{®} type, a per se known product which comprises mineral particles incorporated in a supporting material which can be constituted by cellulose or hemicellulose material or by polymeric material or by a combination of such materials. The mineral particles interact with the electromagnetic radiation by absorption, reflection, refraction, polarization or wavelength shift. These mineral particles can be constituted, for example, by silicon carbide, calcium carbide, titanium dioxide, aluminum oxide, silicon dioxide, zirconium oxide, quartz, etc.

It is known in the literature that by absorbing and reflecting light, the particles increase the oxygen levels in the blood, skin, tissues and muscles, improving body cell oxygenation.

Celliant^{®} has the function of reducing pain caused by arthritis, increasing oxygen levels, facilitating body thermoregulation and inducing the acceleration of healing processes, increasing physical performance and improving sleep quality and wellbeing in general. The main capacity of the product is to absorb and store the emissions of electromagnetic energy originating from the body and to release them so that they can be absorbed by the skin and by muscle tissues. Electromagnetic emissions influence natural biological processes, increasing oxygen levels and improving the body temperature.

The first fabric 11 and the second fabric 12 comprise hemp fiber. This fiber, as is known, is thermally insulating and breathable, and therefore cool in summer and warm in winter. It is a natural fiber that is very resistant to wear and tear, gives fabrics strength and softness at the same time, is known in the literature, shields from electrostatic fields, does not irritate the skin and has antibacterial, antiseptic and anallergenic properties.

The fibers of the first fabric 11 and of the second fabric 12 also comprise carbon.

Copper has antibacterial and antistatic properties, has the capacity to disperse the electrical charges accumulated in the environment and has thermoregulation properties. This fiber, therefore, being antibacterial, is capable of combating germs and fungi. By inhibiting bacterial proliferation it reduces bad odors. Copper is an excellent conductor and therefore copper fiber, as is known in the literature, is useful to discharge the voltages and energies with which the body becomes charged in the course of the day, particularly when living in environments with a high concentration of magnetic waves.

The silver fiber present in the first fabric 11, as is known, has antibacterial properties. Silver is capable of eliminating microbes naturally and contributes to the reduction of bad odors caused by some bacteria, facilitates the healing of wounds, since it facilitates the regeneration of the damaged tissue, and has anti-inflammatory properties. Furthermore, silver is antistatic and thermoregulating, capable of dissipating excess heat keeping the body at an ideal temperature, and silver fiber absorbs and dissipates the electrical charges absorbed from the environment.

As shown in the first two figures, the mat 10 has some stitched seams. In particular, there is a perimetric stitched seam 15 and there is also a series of parallel stitched seams 16, all suitable to mutually join the first fabric 11, the second fabric 12 and the padding 13.

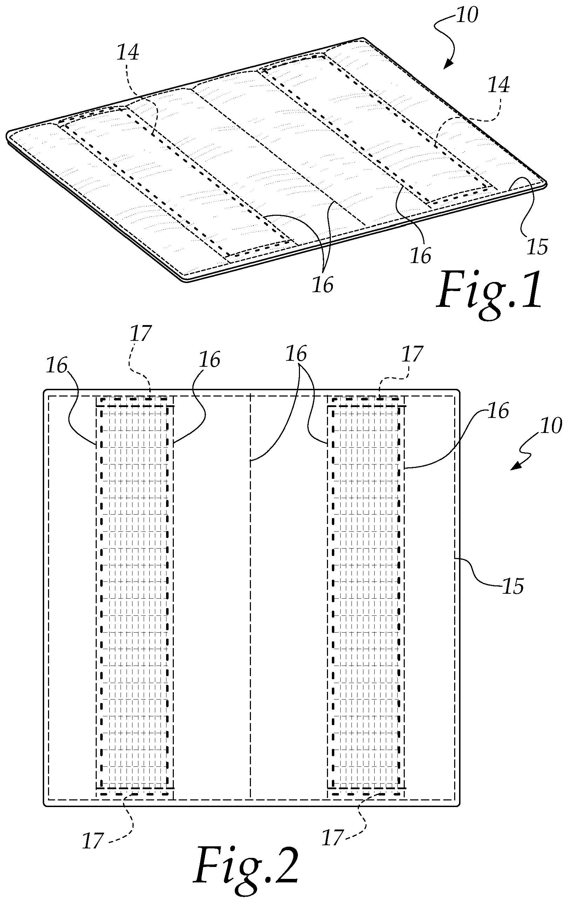

Each magnetic band 14 is arranged between a pair of parallel stitched seams, i.e., two stitched seams 16, and is also sewn to the first fabric 11, the second fabric 12 and the padding 13, transversely proximate to its ends, as indicated in Figures 1 and 2 by the reference numeral 17.

The mat 10 can also comprise a covering sleeve, which is not shown in the illustrated example, preferably with a hook-and-loop closure, in order to make it easy to remove and wash.

The operation of the mat, according to the invention, is evident from what has been described and illustrated and in particular it is evident that the mat uses the combination of the beneficial properties of natural fibers, of Celliant^{®}, and of the bands of magnets, and does not require electricity. The combination of magnets and Celliant^{®} provides an anti-inflammatory, analgesic and biostimulating effect to prevent some pathologies of pets, such as muscle, articular and skeletal problems, while the natural fibers used for the fabrics render the product smell-reducing, antibacterial, antistatic and antimicrobial.

Furthermore, the use of the mat according to the invention is non-invasive and free from side effects.

In practice it has been found that the mat achieves the intended aim and objects, providing a mat for magnetotherapy without electrical cords, the properties of which remain unchanged over time and with smell-reducing, antibacterial, antistatic and antimicrobial characteristics.

The invention thus conceived is susceptible of numerous modifications and variations, all of which are within the scope of the appended claims; all the details may furthermore be replaced with other technically equivalent elements.

In practice, the materials used, so long as they are compatible with the specific use, as well as the contingent shapes and dimensions, may be any according to the requirements and the state of the art.

The disclosures in Italian Patent Application No. 102021000016253 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A mat for animals, **characterized in that** it comprises at least the following layers:
- at least one first fabric (11) containing silver fiber,
- at least one second fabric (12) containing copper fiber,
- at least one padding (13), which contains heat-reactive and/or photocatalytic minerals and is sandwiched between said first fabric (11) and said second fabric (12),
said mat (10) also comprising magnetic bands (14), which are arranged along a same orientation, are mutually spaced and are interposed between said at least one padding (13) and said at least one second fabric (12).

2. The mat according to claim 1, **characterized in that** said padding (13) is of the Celliant^{®} type.

3. The mat according to one or more of the preceding claims, **characterized in that** said magnetic bands (14) are flexible and contain permanent magnets.

4. The mat according to one or more of the preceding claims, **characterized in that** said first fabric (11) and said second fabric (12) comprise hemp fiber.

5. The mat according to one or more of the preceding claims, **characterized in that** the fibers of said first fabric (11) and of said second fabric (12) comprise carbon.

6. The mat according to one or more of the preceding claims, **characterized in that** the distance between said magnetic bands (14) is equal to twice their width.

7. The mat according to one or more of the preceding claims, **characterized in that** said magnetic bands (14) are sewn to said first fabric (11) and said second fabric (12) and to said padding (13).

8. The mat according to one or more of the preceding claims, **characterized in that** each one of said magnetic bands (14) is sewn at least transversely proximate to its ends.

9. The mat according to one or more of the preceding claims, **characterized in that** it has stitched seams (15, 16) adapted to join at least said first fabric (11) and said second fabric (12) and said padding (13).

10. The mat according to one or more of the preceding claims, **characterized in that** it comprises at least one covering sleeve.
